# EUROPÄISCHE PATENTSCHRIFT

(11) **EP 1 262 760 B1**
(45) Veröffentlichungstag und Bekanntmachung des Hinweises auf die Patenterteilung: **13.12.2006**
(21) Anmeldenummer: 02010021.0
(22) Anmeldetag: 04.05.2002
(51) Int. Cl.: G01N 11/02

(54) **Verfahren zum Bestimmen der Viskosität einer Betriebsflüssigkeit einer Brennkraftmaschine**
Method for determining the viscosity of a working fluid of a combustion engine
Méthode de détermination de la viscosité d'in fluide de travail d'un moteur à combustion

(30) Priorität: 22.05.2001 DE 10124888
(43) Veröffentlichungstag der Anmeldung: 04.12.2002
(73) Patentinhaber: Conti Temic microelectronic GmbH, 90411 Nürnberg (DE)
(72) Erfinder: Hubrich, Stefan, 70794 Filderstadt (DE); Pulvermüller, Michael, 73326 Deggingen (DE)

(56) Entgegenhaltungen:
- DE-A- 4 011 448
- DE-A- 19 518 776
- GB-A- 1 529 051
- PATENT ABSTRACTS OF JAPAN vol. 1998, no. 14, 31. Dezember 1998 (1998-12-31) & JP 10 260126 A (KOYO SEIKO CO LTD), 29. September 1998 (1998-09-29)

## Beschreibung

Die Erfindung betrifft ein Verfahren zum Bestimmen der Viskosität einer Betriebsflüssigkeit einer Brennkraftmaschine nach dem Oberbegriff des Anspruchs 1.

Ein derartiges Verfahren ist aus der DE 195 18 776 A1 bekannt. Dort wird ein Verfahren beschrieben, um die Viskosität einer Betriebsflüssigkeit aus dem zeitlichen Verlauf von Änderungen der Füllstandshöhe der Betriebsflüssigkeit zu ermitteln. Bei diesem bekannten Verfahren wird die Viskosität des Motorenöls nach dem Abschalten einer Brennkraftmaschine durch Messung des zeitlichen Verlaufs der Füllstandshöhe des Motorenöls bestimmt. Die Rückkehr des Motorenöls in das Vorratsvolumen erfolgt mit einer Zeitverzögerung nach dem Abstellen des Motors in Abhängigkeit von der Viskosität. Neben dem zeitlichen Verlauf der Füllstandshöhe findet auch die Temperatur des Motorenöls Eingang in das bekannte Verfahren, da die Viskosität des Motorenöls im allgemeinen von seiner Temperatur abhängig ist.

Neben diesen auf einfache Weise zu messenden Größen wird die Messung der Viskosität nach dem bekannten Verfahren jedoch auch von weiteren Größen beeinflusst. So kann eine hohe dynamische Beanspruchung der Brennkraftmaschine in einem Zeitraum unmittelbar vor dem Abstellen zu einer Verschäumung des Motorenöls führen. Die daraus resultierende langsamere Änderung der Füllstandhöhe nach dem Abschalten verfälscht dann die eigentliche Messung. Eine objektive Messgröße für die dynamische Beanspruchung der Brennkraftmaschine ist aber nicht auf einfache Weise zu bestimmen.

Ein Abstellen der Brennkraftmaschine bei Schräglage des Fahrzeugs beeinträchtigt ebenfalls das Zurückfließen des Motorenöls in das Vorratsvolumen.

Ein weiterer Nachteil ist darin zu sehen, dass die für die Messung erforderlichen Elektronikmodule noch die erforderliche Zeit nach dem Abstellen der Brennkraftmaschine aktiv betrieben werden müssen.

Aufgabe der Erfindung ist es, ein Verfahren zum Bestimmen der Viskosität einer Betriebsflüssigkeit einer Brennkraftmaschine mit Hilfe der Messung der Füllstandshöhe anzugeben, das mit wenigen Messgrößen ein genaues Ergebnis liefert und das während des Betriebs der Brennkraftmaschine abläuft.

Diese Aufgabe wird durch ein Verfahren mit den Merkmalen des Anspruchs 1 gelöst. Die Ausgestaltung der Erfindung erfolgt gemäß den Merkmalen der abhängigen Ansprüche.

Bei dem Verfahren zum Bestimmen der Viskosität einer Betriebsflüssigkeit einer Brennkraftmaschine eines Fahrzeugs wird zunächst ein erstes Signal, das dem zeitlichen Verlauf der Füllstandshöhe entspricht, und ein zweites Signal erfasst, das dem zeitlichen Verlauf eines Zustands des Fahrzeugs entspricht, das Einfluss auf die gemessene Füllstandshöhe der Betriebsflüssigkeit hat. Das erste und das zweite Signal werden anschließend einer Filterung zugeführt, die eine Bewertung der Signale hinsichtlich ihrer Dynamik erlaubt. Als Ergebnis werden ein drittes und ein viertes Signal erzeugt, wobei das dritte Signal Ergebnis der Filterung des ersten und das vierte Signal Ergebnis der Filterung des zweiten ist. Anschließend wird das vierte Signal mit einem Schwellwert verglichen und in Abhängigkeit vom Ergebnis des Vergleichs ein Verhältnis des vierten Signals zum dritten Signal gebildet. Der Mittelwert über eine Vielzahl von Werten des Verhältnisses des vierten Signals zum dritten Signal entspricht der Viskosität der Betriebsflüssigkeit.

Der Vorteil der Erfindung besteht darin, bereits verfügbare Sensorsignale von in der Serie befindlichen, erprobten Sensoranordnungen miteinander zu kombinieren und daraus als zusätzliche Kenngröße die Viskosität zu ermitteln. Die hierfür notwendige Rechenleistung wird von Standardsteuergeräten übernommen.

In einer vorteilhaften Ausführungsform des Verfahrens wird als zweites Signal die Querbeschleunigung des Fahrzeugs erfasst wird. Das kontinuierliche Messsignal des Ölstandssensors wird mit dem kontinuierlichen Signal des Querbeschleunigungssensors verknüpft. Das Ergebnis führt zu einer Aussage über die Viskosität des Öles. Querbeschleunigungen werden für andere Systeme wie z.B. ESP regelmäßig erfasst.

Die Erfassung der Messwerte des ersten und zweiten Signals erfolgt vorzugsweise mit einer Rate von ca. 1 bis 1,5/s. Diese Messrate ist ausreichend groß um verlässliche Aussagen zur Viskosität zu machen und ausreichend klein um nicht zu große Datenmengen zu erzeugen.

Zur Filterung des ersten bzw. zweiten Signals wird vorzugsweise eine gleitende Standardabweichung über eine erste Anzahl von Werten gebildet. Das dritte bzw. vierte Signal bilden den Ausgang der Filterung. Die gleitende Standardabweichung liefert mit relativ einfachen statistischen Methoden eine gute Aussage über die Dynamik der Signale.

Dabei liegt die Anzahl der Werte zur Ermittlung der gleitenden Standardabweichung im Bereich von 5 bis 100. Ein Wert von 15 hat sich bei einer Reihe Messungen als besonders vorteilhaft herausgestellt.

Die Bildung des Mittelwerts des Verhältnisses des vierten Signals zum dritten Signal erfolgt Vorteilhafterweise über ca. 50 bis 300 Werte.

Die Erfindung soll nachfolgend anhand von Ausführungsbeispielen und Figuren näher erläutert werden. Kurze Beschreibung der Figuren:
- Figur 1: zeigt ein Diagramm mit dem zeitlichen Verlauf von Öltemperatur, Querbeschleunigung und Füllstandshöhe einer Testfahrt.
- Figur 2: zeigt ein Diagramm mit dem zeitlichen Verlauf von Öltemperatur, SNV(Q), SNV(H) und ermittelter Viskosität X der Testfahrt.
- Figur 3: zeigt ein Ablaufdiagramm des Verfahrens.

Eine besonders vorteilhafte Ausführung des Verfahrens betrifft die Bestimmung der Viskosität eines Motors eines Kraftfahrzeugs.

Der bereits heute in Serie laufende QLT-Ölstandssensor der Firma Temic liefert ein kontinuierliches Füllstandssignal.

Die Information der vorliegenden Querbeschleunigung liefert ein Querbeschleunigungssensor des ESP- oder Fahrwerksregelungssystems.

Aufgrund der Dynamik des Öles in der Ölwanne ändert sich das Füllstandssignal in Abhängigkeit des Fahrzustandes, insbesondere bei Querbeschleunigungen bewirkt die Zentrifugalkraft einen unterschiedlichen momentanen Füllstand auf beiden Seiten der Ölwanne. Diese dynamischen Füllstandsunterschiede sind mit abnehmender Viskosität (dünnflüssiger) stärker ausgeprägt, d.h. zähes Öl zeigt weniger ausgeprägte Füllstandsunterschiede bei Kurvenfahrt wie dünnflüssiges Öl. Führt man beide Sensorinformationen zusammen z.B. im Motorsteuergerät, so lässt sich eine definierte Auswertung der Füllstandsänderung in Abhängigkeit der Querbeschleunigung machen, was eine Aussage über die Zähigkeit des Öles =Viskosität ermöglicht. Durch Nutzung der im Fahrzeug bereitgestellten Sensorsignale des Querbeschleunigungssensors und des QLT-Ölstandssensor lässt sich eine bisher nicht erfassbare zusätzliche Qualitätskenngröße (= Viskosität) ermitteln.

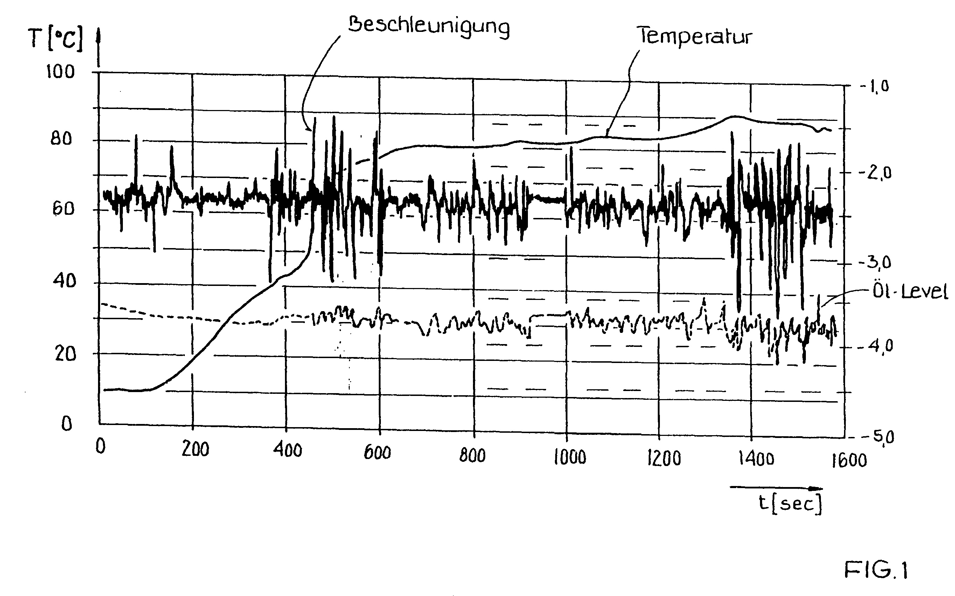

Figur 1 zeigt ein Diagramm mit dem zeitlichen Verlauf von Öltemperatur, Querbeschleunigung und Füllstandshöhe einer Testfahrt. Zu Beginn der Messung erkennt man, dass bei niederen Temperaturen die Viskosität des Motorenöls noch so hoch ist, dass auch große Querbeschleunigungen nur einen kaum wahrnehmbaren Einfluss auf die gemessene Füllstandshöhe ausüben. Dieser Einfluss wird mit steigender Temperatur des Motorenöls immer größer und ist dann schließlich bei normaler Betriebstemperatur deutlich ausgeprägt.

Um nun die beiden Signale in Relation zueinander zu setzen, werden sie zunächst einer Filterung zugeführt, die als Ergebnis ein Maß für die Dynamik der beiden Signale liefert. Unter Filterung ist in diesem Zusammenhang ganz allgemein jede Signalaufbereitung zu verstehen, die eine Bewertung hinsichtlich der Dynamik erlaubt.

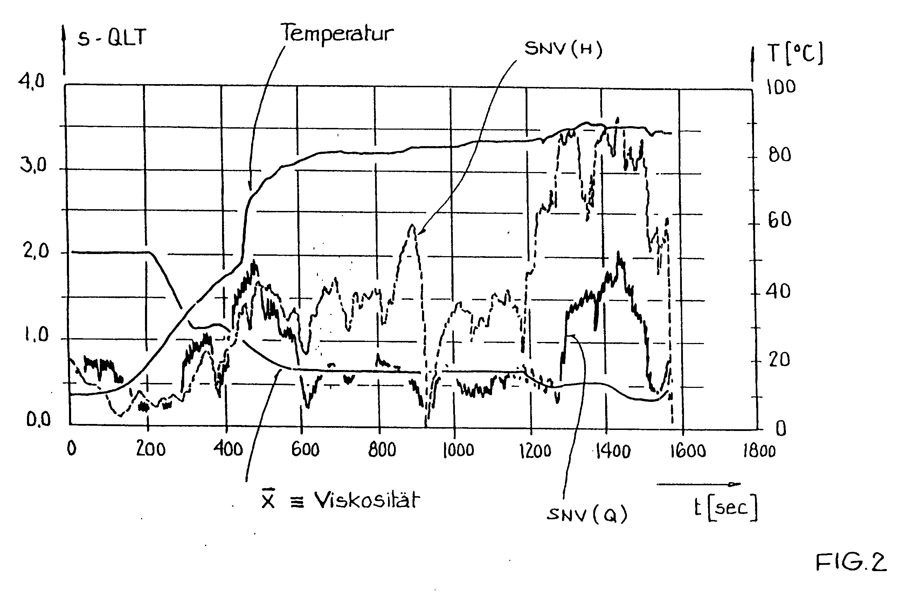

Als vorteilhaft hat sich dafür die gleitende Standardnormalverteilung SNV über eine feste Anzahl von Messwerten erwiesen. In der Figur 2 sind die Verläufe der entsprechend gefilterten Signale der Querbeschleunigung SNV(Q) und der gemessenen Füllstandshöhe SNV(H) analog zur Figur 1 aufgetragen.

Anschließend werden die gefilterten Signale zueinander ins Verhältnis gesetzt, sobald der Wert für das Signal, dessen Größe für die Änderung der Füllstandshöhe ursächlich verantwortlich ist - hier die Querbeschleunigung -, über einem vorgegebenen Schwellwert K liegt. Der Mittelwert über eine Vielzahl dieser Verhältniswerte entspricht im wesentlichen der Viskosität des Motorenöls.

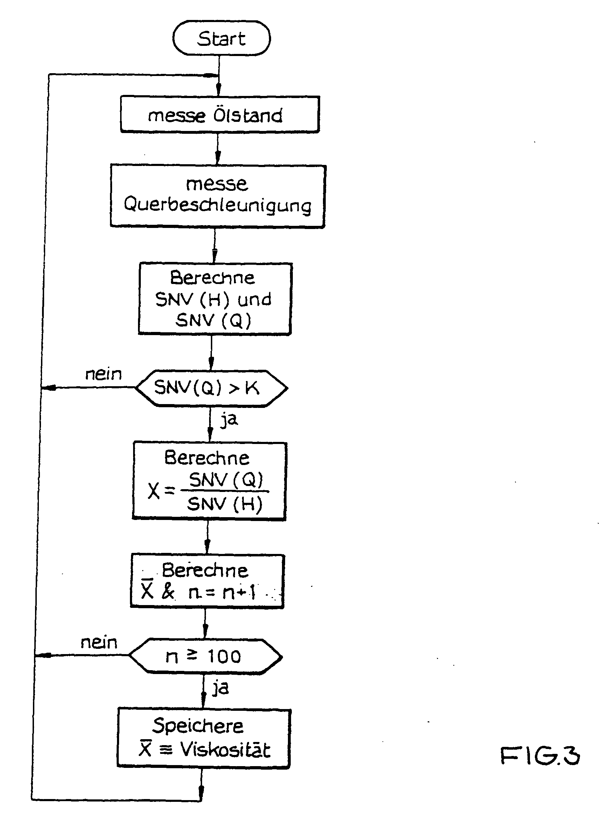

Die Figur 3 zeigt ein Ablaufdiagramm des Verfahrens. Zu Beginn erfolgt die Messung des Ölstands H und der Querbeschleunigung Q. Hierbei hat es sich als ausreichend erwiesen, wenn die Abtastung der Signale, bzw. die Erfassung der Messwerte mit einer Rate von ca. 1 bis 1,5/s erfolgt. Anschließend wird die gleitende Standardnormalverteilung SNV für beide Signale SNV(Q), SNV(H) über eine vorgegebene Anzahl von n Werten berechnet. Die Anzahl n = 15 hat sich hierbei als vorteilhaft erwiesen. In der Praxis kann n je nach Anwendung aus einem weiten Bereich von ca. 5 bis 100 ausgewählt werden. Dann wird die gleitende Standardnormalverteilung der Querbeschleunigung SNV(Q) mit einem Schwellwert K verglichen. Wird der Schwellwert K nicht überschritten erfolgt ein Sprung zurück zum Anfang des Verfahrens; wird der Schwellwert K überschritten, wird der Quotient X = SNV(Q)/SNV(H) berechnet. Wurden bereits zuvor Werte für X ermittelt, wird abschließend der Mittelwert *X̅* errechnet. Sobald der Mittelwert *X̅* über eine genügend hohe Anzahl von Einzelwerten gebildet wurde kann er als Kenngröße für die Viskosität verwendet werden. Im Ablaufdiagramm der Figur 3 ist dies am Beispiel einer Zählvariablen dargestellt, die den Wert 100 erreichen muss, bevor der Mittelwert *X̅* als Kenngröße für Viskosität des Motorenöls abgespeichert wird.

Die Berechnung der Viskosität kann durch eine entsprechende Anordnung auch direkt im Ölstandsensor erfolgen, wenn dort auch auf die Daten des Beschleunigungssensors zugegriffen werden kann. Im Normalfall werden die Daten in einem zentralen Steuergerät erfasst und ausgewertet. Die berechnete Viskosität wird schließlich über eine Schnittstelle an ein Diagnosegerät gesendet oder von einer Onboard-Diagnose-Einrichtung im Kraftfahrzeug ausgewertet.

Neben der Querbeschleunigung können zur Ermittlung der Viskosität noch eine Reihe alternativer Größen berücksichtigt werden, solange sie nur eine Auswirkung auf die gemessene Füllstandshöhe der Betriebflüssigkeit haben. So kann beispielsweise auch die Längsbeschleunigung oder die Motorendrehzahl anstelle der Querbeschleunigung als Ausgangsgröße verwendet werden. Beide Größe bewirken gleichermaßen eine Änderung der Füllstandshöhe des Motorenöls, wobei ihr Einfluss auf die Änderung wiederum von der Viskosität abhängig ist. Bei der Verwendung des Signals für die Längsbeschleunigung wird analog zur Querbeschleunigung durch Bewegung des Öls in der Ölwanne die gemessene Füllstandshöhe beeinflusst. Bei der Verwendung des Signals für die Drehzahl als Ausgangsgröße kann der Umstand genutzt werden, dass sich die im Ölkreislauf befindliche Ölmenge in Abhängigkeit von der Drehzahl ändert. Es müssen lediglich die Signalerfassung und die Mess- und Filterparameter entsprechend angepasst werden, um auch aus diesen Größen die Viskosität des Öls in analoger Weise bestimmen zu können.

## Patentansprüche

1. Verfahren zum Bestimmen der Viskosität einer Betriebsflüssigkeit einer Brennkraftmaschine eines Fahrzeugs durch Ermitteln der Füllstandshöhe der Betriebsflüssigkeit, wobei ein erstes Signal erfasst wird, das dem zeitlichen Verlauf der Füllstandshöhe entspricht,
**dadurch gekennzeichnet,**
• **dass** ein zweites Signal erfasst wird, das dem zeitlichen Verlauf eines Zustands des Fahrzeugs entspricht, das Einfluss auf die gemessene Füllstandshöhe der Betriebsflüssigkeit hat,
• **dass** das erste und das zweite Signal einer Filterung zugeführt werden, die eine Bewertung der Signale hinsichtlich ihrer Dynamik erlaubt und als Ergebnis ein drittes und ein viertes Signal liefert,
• **dass** das vierte Signal mit einem Schwellwert verglichen und in Abhängigkeit vom Ergebnis des Vergleichs ein Verhältnis des vierten Signals zum dritten Signal gebildet wird und
• **dass** der Mittelwert über eine Vielzahl von Werten der Viskosität der Betriebsflüssigkeit entspricht.

2. Verfahren nach Anspruch 1, **dadurch gekennzeichnet, dass** als zweites Signal die Querbeschleunigung des Fahrzeugs erfasst wird.

3. Verfahren nach Anspruch 1 oder 2, **dadurch gekennzeichnet, dass** die Erfassung der Messwerte des ersten und zweiten Signals mit einer Rate von ca. 1 bis 1,5/s erfolgt.

4. Verfahren nach einem der Ansprüche 1 bis 3, **dadurch gekennzeichnet, dass** zur Filterung eine gleitende Standardabweichung über eine erste Anzahl von Werten des ersten bzw. zweiten Signals gebildet und als drittes bzw. viertes Signal den Ausgang des Filters bilden.

5. Verfahren nach Anspruch 4, **dadurch gekennzeichnet, dass** die Anzahl der Werte zur Ermittlung der gleitenden Standardabweichung im Bereich von 5 bis 100 liegt.

6. Verfahren nach einem der Ansprüche 1 bis 5, **dadurch gekennzeichnet, dass** Mittelwert des Verhältnisses des vierten Signals zum dritten Signal über ca. 50 bis 300 Werte gebildet wird.

## Claims

1. A procedure for calculating the viscosity of an oil filling level in an internal combustion engine of a vehicle by determining the filling level of the oil filling level, whereby a first signal is recorded which corresponds to the time progression of the filling level,
**characterized in that**
• a second signal is recorded, which corresponds to the time progression of a state of the vehicle which influences the measured filling level of the oil filling level
• the first and the second signal are fed to a filter, which enables the signals to be evaluated with regard to their dynamic, and which supplies as a result a third and a fourth signal
• the fourth signal is compared with a threshold value and depending on the result of the comparison, a ratio between the fourth signal and the third signal is formed, and
• the average value corresponds to the viscosity of the oil filling level via a plurality of values

2. A procedure according to claim 1, **characterized in that** the lateral acceleration of the vehicle is recorded as the second signal.

3. A procedure according to either of claims 1 or 2, **characterized in that** the recording of the measured values of the first and second signal is conducted at a rate of approx. 1 to 1.5/s.

4. A procedure according to any one of claims 1 to 3, **characterized in that** for filtering purposes, a sliding standard deviation is formed via a first quantity of values from the first or second signal, and as the third or fourth signal, forms the output of the filter.

5. A procedure according to claim 4, **characterized in that** the quantity of values for determining the sliding standard deviation lies in the range of 5 to 100.

6. A procedure according to any one of claims 1 to 5, **characterized in that** the average value of the ratio between the fourth signal and the third signal is formed via approx. 50 to 300 values.

## Revendications

1. Procédé de détermination de la viscosité d'un liquide de service d'un moteur à combustion interne d'un véhicule par la détermination du niveau du liquide de service, un premier signal étant détecté qui correspond à l'allure dans le temps du niveau,
**caractérisé en ce que**
• un deuxième signal est détecté, qui correspond à l'allure dans le temps d'un état du véhicule, qui exerce une influence sur le niveau mesuré du liquide de service,
• le premier signal et le deuxième signal sont conduits à un filtrage qui permet une évaluation des signaux quant à leur dynamique et fournit comme résultat un troisième signal et un quatrième signal,
• le quatrième signal est comparé à une valeur de seuil et, en fonction du résultat de la comparaison, un rapport du quatrième signal au troisième signal est formé, et
• la valeur moyenne sur une multiplicité de valeurs correspond à la viscosité du liquide de service.

2. Procédé selon la revendication 1, **caractérisé en ce que**, en tant que deuxième signal, l'accélération transversale du véhicule est détectée.

3. Procédé selon la revendication 1 ou 2, **caractérisé en ce que** la détection des valeurs de mesure du premier signal et du deuxième signal est effectuée à une cadence d'environ 1 à 1,5/s.

4. Procédé selon une des revendications 1 à 3, **caractérisé en ce que**, pour le filtrage, il est formé un écart type mobile sur un première nombre de valeurs du premier signal ou du deuxième signal, et le troisième et le quatrième signal forment la sortie du filtrage.

5. Procédé selon la revendication 4, **caractérisé en ce que** le nombre des valeurs pour déterminer l'écart type mobile se situe dans la plage de 5 à 100.

6. Procédé selon une des revendications 1 à 5, **caractérisé en ce que** la valeur moyenne du rapport du quatrième signal au troisième signal est formée sur environ 50 à 300 valeurs.
